# EUROPÄISCHE PATENTSCHRIFT

(11) **EP 1 075 457 B1**
(45) Veröffentlichungstag und Bekanntmachung des Hinweises auf die Patenterteilung: **13.03.2002**
(21) Anmeldenummer: 98929281.8
(22) Anmeldetag: 04.05.1998
(51) Int. Cl.: C07C 17/38

(54) **VERFAHREN ZUR AUFARBEITUNG DER SPALTPRODUKTE AUS DER THERMISCHEN SPALTUNG VON 1,2-DICHLORETHAN**
METHOD FOR PREPARING DECOMPOSITION PRODUCTS FROM THE THERMAL DECOMPOSITION OF 1,2-DICHLOROETHANE
PROCEDE POUR LE RETRAITEMENT DES PRODUITS DE DECOMPOSITION ISSUS DE LA DECOMPOSITION THERMIQUE DE 1,2-DICHLOROETHANE

(43) Veröffentlichungstag der Anmeldung: 14.02.2001
(73) Patentinhaber: Vinnolit Monomer GmbH & Co. KG, 85737 Ismaning (DE)
(72) Erfinder: WILD, Thomas, D-84508 Burgkirchen (DE); EICHLER, Juergen, D-84556 Kastl (DE); STRANG, Werner, D-84508 Burgkirchen (DE); WIDMANN, Peter, D-84508 Burgkirchen (DE)
(74) Vertreter: Forstmeyer, Dietmar, Dr. rer. nat., Dipl.-Chem.
(86) Internationale Anmeldenummer: EP9802611
(87) Internationale Veröffentlichungsnummer: WO9957088

(56) Entgegenhaltungen:
- EP-A- 0 073 941
- DE-A- 4 033 047
- DE-A- 4 132 761
- DE-A- 4 132 762
- DE-A- 4 139 632

## Beschreibung

Die technische Herstellung von Vinylchlorid (im folgenden "VC") erfolgt durch thermische Spaltung von 1,2-Dichlorethan (im folgenden "EDC") bei Temperaturen von 450 bis 600 °C unter erhöhtem Druck. Hierbei werden etwa 40 bis 65 % des eingesetzten EDC in VC und Chlorwasserstoff gespalten. Bei dieser Spaltung entstehen Nebenprodukte mit höherem und niedrigerem Siedepunkt als das EDC. Es muß somit eine mehrstufige destillative Auftrennung erfolgen.

Die gasförmigen Produkte der thermischen Spaltung (Spaltgas) werden zunächst zur Abtrennung von gröberen Kokspartikeln durch ein Filter geleitet, kondensiert und in die erste Destillationskolonne (HCl-Kolonne) geleitet, in der der Chlorwasserstoff als Kopfprodukt abdestilliert wird. In einer weiteren Kolonne (VC-Kolonne) wird das VC als Kopfprodukt abdestilliert. Der Sumpf dieser Kolonne enthält das nichtumgesetzte EDC sowie leichter und höher siedende Nebenprodukte und feinere Kokspartikel.

Diese Kokspartikel stören insbesondere bei der erforderlichen Abtrennung des Benzols, so daß bisher eine aufwendige Aufteilung des Sumpfproduktes und Reinigung des Teilstroms erforderlich wurde, der der Benzolabtrennung zugeführt wurde. Bevorzugt erfolgte diese Aufteilung und Teilreinigung durch eine Entspannungsverdampfung.

Aus der DE-A-40 33 047 ist ein Verfahren zur Benzolabtrennung aus dem bei der VC-Herstellung zurückgewonnenen EDC, nach der Abtrennung des VC und Chlorwasserstoffs, bekannt, das dadurch gekennzeichnet ist, daß man das EDC aus dem Kolonnensumpf der VC-Kolonne in einen von hochsiedenden Substanzen gereinigten und einen ungereinigten Produktstrom aufteilt, den gereinigten Produktstrom in Gegenwart von metallischem Eisen bei einer Temperatur von 30 bis 85 °C mit Chlor behandelt und diesen Produktstrom mit dem ungereinigten Produktstrom wieder vereinigt und gemeinsam in einem weiteren Reinigungsschritt das EDC von Hochsiedern abtrennt. Eine Verbesserung dieses Verfahrens besteht darin, daß als Katalysator γ-Aluminiumoxid eingesetzt wird (DE-A-41 29 391).

Aus der DE-A-41 39 632 ist weiterhin ein Verfahren zur Benzolabtrennung aus dem bei der VC-Herstellung zurückgewonnenen EDC nach der Abtrennung des VC und Chlorwasserstoffs bekannt, das dadurch gekennzeichnet ist, daß man das verunreinigte EDC aus dem Kolonnensumpf der VC-Kolonne in einen von hochsiedenden Substanzen (Kp₇₆₀ > 83,7 °C) befreiten Produktstrom I und einen die hochsiedenden Substanzen (Kp₇₆₀ > 83,7 °C) enthaltenden Produktstrom II trennt, den Produktstrom I in Gegenwart von metallischem Eisen bei einer Temperatur von 30 bis 85 °C mit Chlor behandelt und den behandelten Produktstrom I mit dem Produktstrom II vereinigt und die vereinigten Produktströme gemeinsam bei einer Temperatur von 30 bis 85 °C mit Chlor behandelt und aus diesem Produktstrom in einer Destillationszone gereinigtes EDC abtrennt.

Aus der DE-A-41 32 761 ist ein Verfahren zur Aufarbeitung des Spaltgases, welches bei der thermischen Spaltung von EDC zu VC mit einer Temperatur von 480 bis 540 °C und einem Druck von 15 bis 25 bar anfällt und gegebenenfalls in einer Wärmerückgewinnung auf eine Temperatur von 180 bis 280 °C gekühlt und mit dieser Temperatur in eine Quenchzone geleitet wird, in der es mit kondensiertem Spaltgas gekühlt und gewaschen wird, bekannt, das dadurch gekennzeichnet ist, daß man a) 80 bis 99 Gew.-% der abgekühlten Spaltgase gasförmig als Kopfprodukt und 1 bis 20 Gew.-% der abgekühlten Spaltgase als flüssiges Sumpfprodukt der Quenchzone entnimmt, b) den im Sumpfprodukt der Quenche enthaltenen Koks zerkleinert, c) die hierbei erhaltene Dispersion in einer Abtreibezone in ein Destillat und einen Sumpf trennt und den Sumpf aus dem Verfahren ausschleust und d) das Destillat der Abtreibezone mit dem gasförmigen Kopfprodukt der Quenchzone nach dessen Kondensation vereinigt. Bevorzugt werden 3 bis 6 Gew.-% als Sumpfprodukt aus der Quenchzone entnommen.

Es wurde nun gefunden, daß sich die folgende Bètriebsweise der Quenche dazu eignet, die Benzolchlorierung erheblich zu vereinfachen: Es kann auf die Aufteilung der Produktströme und die Reinigung eines dieser Produktströme dann verzichtet werden, wenn man die gasförmigen Produkte aus der thermischen Spaltung von EDC auf einen relativ engen, niedrigen Temperaturbereich von etwa 150 bis 180 °C, vorzugsweise 160 bis 170 °C, abkühlt, so daß in dieser Kondensationsstufe nur ein geringer Anteil des EDC zusammen mit hochsiedenden Anteilen und gebildetem Koks kondensiert. Der größte Teil des nichtumgesetzten EDC wird somit mit den Produkten VC und Chlorwasserstoff in die destillative Aufarbeitung geleitet, wobei - in an sich bekannter Weise - zunächst der Chlorwasserstoff und in einer weiteren Stufe das VC vom EDC destillativ abgetrennt werden. Das zurückbleibende EDC kann nun - ohne Reinigung - in die Anlage zur Entfernung des Benzols geführt werden, die zweckmäßig nach dem Verfahren der DE-A-41 29 391 mit γ-Aluminiumoxid erfolgt.

Im allgemeinen genügt es, nur einen Anteil des EDC der Benzolchlorierung zu unterziehen, um einen hinreichend geringen Anteil an Benzol und dessen Folgeprodukten im EDC beziehungsweise VC zu erhalten. Zur Aufteilung in Teilströme genügt aber erfindungsgemäß eine einfache Vorrichtung wie ein Regelventil

Erfindungsgemäß können weniger als 7 %, vorzugsweise weniger als 5 %, des EDC kondensiert werden, so daß nur ein relativ geringer Anteil an kokshaltigem Kondensat getrennt aufgearbeitet werden muß. Weiterhin entfällt die bei den bekannten Benzolchlorierungsverfahren erforderliche Entspannungsverdampfung, so daß das erfindungsgemäße Verfahren wesentlich weniger aufwendig ist.

Die Erfindung wird in dem folgenden Beispiel näher erläutert.

### Beispiel

Das Spaltgas aus der EDC-Spaltung wird mit Spaltgas-Kondensat in der Quenche auf etwa 160 bis 170 °C gekühlt, wobei 4 % des Spaltgases kondensieren. Im Kondensat findet sich der gesamte Koks. Dieser wird abgezogen und getrennt aufgearbeitet.

Die nichtkondensierten etwa 96 % des Spaltgases gehen gasförmig über Kopf der Quenche ab und werden zur Zwischenkondensation geleitet und dort kondensiert. Dieses Kondensat wird ebenfalls in die HCl-Kolonne geleitet. Bei der anschließenden Destillation in der HCl- und VC-Kolonne sind die Kolonnensümpfe koksfrei. Der Sumpfstrom aus der VC-Kolonne wird nach Abkühlung auf etwa 30 bis 50 °C mit einem Regelventil in zwei Teilströme aufgeteilt: Etwa 8 t/Stunde gehen in die Benzolchlorierung, etwa 20 t/Stunde werden am Benzolchlorierungsreaktor vorbeigeführt und mit dem Produkt aus diesem Reaktor vereinigt. Der vereinigte Produktstrom wird ohne Kühlung zur Chloropren-Chlorierung gefördert und gelangt von dort zur Hochsiederkolonne.

Bei der erfindungsgemäßen Verfahrensweise entfallen somit die mit der Entspannung und der Filtration verbundenen Anlageteile.

## Patentansprüche

1. Verfahren zur Aufbereitung der Spaltprodukte aus der thermischen Spaltung von 1,2-Dichlorethan, **dadurch gekennzeichnet, daß** die gasförmigen Produkte auf einen Temperaturbereich von 150 bis 180°C abgekühlt werden, der nichtkondensierte überwiegende Anteil, bestehend aus den Spaltprodukten HCl und VC und dem nicht-umgesetzten 1,2-Dichlorethan, der destillativen Aufarbeitung zugeführt wird und das nicht-umgesetzte 1,2-Dichlorethan ohne weitere Reinigung der Benzolchlorierung zugeführt und der untergeordnete Anteil, der den gesamten Koks enthält, einer separaten Aufarbeitung unterzogen wird.

2. Verfahren nach Anspruch 1, **dadurch gekennzeichnet, daß** die Abkühlung auf einen Temperaturbereich von 160 bis 170°C erfolgt.

3. Verfahren nach Anspruch 1 oder 2, **dadurch gekennzeichnet, daß** weniger als 7 % des Spaltgases kondensiert werden.

4. Verfahren nach einem oder mehreren der vorhergehenden Ansprüche, **dadurch gekennzeichnet, daß** nur ein Teilstrom des nicht-umgesetzten 1,2-Dichlorethans der Benzolchlorierung zugeführt wird.

## Claims

1. Process for working up the cracking products from the thermal cracking of 1,2-dichloroethane, **characterized in that** the gaseous products are cooled to a temperature range from 150 to 180°C, the uncondensed predominant proportion, consisting of the cracking products HCl and VC and the unreacted 1,2-dichloroethane is fed to the work-up by distillation and the unreacted 1,2-dichloroethane is fed without further purification to the benzene chlorination and the subsidiary proportion which comprises all of the coke is subjected to a separate work-up.

2. Process according to Claim 1, **characterized in that** the cooling is carried out to a temperature range from 160 to 170°C.

3. Process according to Claim 1 or 2, **characterized in that** less than 7% of the cracking gas is condensed.

4. Process according to one or more of the preceding claims, **characterized in that** only one partial stream of the unreacted 1,2-dichloroethane is fed to the benzene chlorination.

## Revendications

1. Procédé pour préparer des produits de dissociation provenant de la dissociation thermique du 1,2-dichloroéthane, **caractérisé en ce que** les produits gazeux sont refroidis jusqu'à une plage de températures de 150 à 180°C, la proportion prépondérante non condensée constituée des produits de dissociation HCl et VC et du 1,2-dichloroéthanne n'ayant pas réagi, alimente le traitement par distillation et le 1,2-dichloroéthane n'ayant pas réagi alimente la chloration du benzène sans purification supplémentaire et la proportion secondaire qui contient tout le coke, est soumise à un traitement séparé.

2. Procédé selon la revendication 1, **caractérisé en ce que** le refroidissement a lieu jusqu'à une plage de températures de 160 à 170°C.

3. Procédé selon la revendication 1 ou 2, **caractérisé en ce que** moins de 7% du gaz de dissociation sont condensés.

4. Procédé selon l'une ou plusieurs des revendications précédentes, **caractérisé en ce que** seulement un courant partiel du 1,2-dichloroéthanne n'ayant pas réagi, alimente la chloration du benzène.
